# EUROPEAN PATENT APPLICATION

(11) **EP 0 710 485 A1**
(43) Date of publication of application: **08.05.1996**
(21) Application number: 95306324.5
(22) Date of filing: 11.09.1995
(51) Int. Cl.: A61K 38/00, A23L 1/305, A61K 31/415

(54) **use of anserine in the manufacture of a medicament for stimulating hematopoiesis**

(30) Priority: 09.09.1994 JP 19940215813; 09.09.1994 JP 19940216236
(71) Applicant: SUNTORY LIMITED, Kita-ku, Osaka-shi, Osaka (JP)
(72) Inventor: Harada, Masami, Mishima-gun, Osaka (JP); Yang, Zhi-bo, Otsu-shi, Shiga (JP)
(74) Representative: Stoner, Gerard Patrick, et al

(57) **Abstract**

A hemopoietic function stimulating composition, or a preventing or alleviating composition for medical symptoms caused by the decrease of the number of erythrocytes, comprising at least one imidazole compound selected from the group consisting of anserine, balenine, τ-methylhistidine, τ-methylhistidine, carnosine and/or homocarnosine.

## Description

The present invention relates a hemopoietic function-stimulating agent, foods or beverages exhibiting stimulated hemopoietic functions and the processes for the production thereof, as well as a pharmaceutical composition and foods or beverages having preventive or alleviating actions against medical symptoms caused by a decrease in the number of erythrocytes.

### BACKGROUND

Recently, major clinical problems have appeared such that, since therapies such as anticancer agents and radiation therapy inhibit hemopoietic functions causing a decrease in the number of leukocytes or erythrocytes, then anemia is caused, and the drug therapy and radiation therapy cannot be continued. Although transplantation of bone marrow cells could be useful for such disorders of hemopoietic functions, since the availability of fresh bone marrow cells is limited and there is risk of contamination by viruses, then a safe hemopoietic function stimulating agent capable of being orally administrated is sought. In addition, serum iron and hemoglobin contents decrease after blood donation, operation, and childbirth, and it takes a long time for the patient to recover to normal values and it is sought to shorten the recovery period.

L-anserine (β-alanyl-π-methyl-L-histidine), L-balenine (β-alanyl-π-methyl-L-histidine) and L-carnosine (β-alanyl-L-histidine) are known substances which are dipeptides present in muscle tissues of mammals, fowls, reptiles, amphibians etc. Homocarnosine is a known peptide mainly present in the brain. In addition, π-methyl histidine and τ-methyl histidine are known as intermediates for synthesis of anserine and balenine respectively Since these dipeptides were found, at the beginning of this century, various research has been carried out, and it was reported that anserine, balenine and carnosine are present in the muscles of vertebrates in a concentration of 1 to 20 mM.

Although it is known that their content varies dependent on the kinds of the muscles, animals, age etc., their physiological actions have not yet been completely clarified. However, their functions are still being investigated, and Japanese Unexamined Patent Publication (Kokai) Nos. 61-246218 and 61-186322 disclose that L-anserine and L-carnosine recover an abnormal lowering of immune response and suppresses its excessive acceleration so as to maintain normal functions of the immune system. Moreover, Japanese Unexamined Patent Publication (Kokai) No. 4-16,166 discloses the actions of carnosine, anserine, and balenine to suppress an increase in blood pressure, and Japanese Unexamined Patent Publication (Kokai) No. 4-187,610 discloses the actions of carnosine, anserine and balenine to increase the flexibility and softness of the skin.

On the other hand, to inhibit the decrease of leukocytes caused by the side-effects of chemotherapy and radiation therapy which inhibit the proliferation of cancer and viruses, Japanese Unexamined Patent Publication (Kokai) No. 1-93,526 discloses the effectiveness of homocarnosine and Japanese Examined Patent Publication (Kokoku) No. 5-13,928 discloses the effectiveness of L-carnosine. In addition it is reported that when a mouse administered with carnosine is irradiated, the viability of the mouse increases and the activity of colony formation by hematopoietic stem cells also increases (Bull. Exp. Biol. Med. 112 (7) 966-968 (1991)).

In addition, Japanese Unexamined Patent Publication (Kokai) No. 4-187,067 describes that carnosine, anserine and balenine have the effect of preventing oxidation of proteinaceous foods, and Japanese Examined Patent Publication (Kokoku) No. 63-47,435 describes that when carnosine, anserine or balenine is added to a product to a total amount of at least 0.1% and pH value is adjusted to at least 6.5, the taste of the product is improved. In addition, Japanese Unexamined Patent Publication (Kokai) No. 2-35057 discloses a functional food containing a high concentration of L-carnosine.

However, we-have now found that anserine, balenine, τ-methylhistidine and τ-methylhistidine have hemopoietic function-stimulating actions, and that anserine, carnosine, balenine, homocarnosine, τ-methylhistidine and τ-methylhistidine have an action to alleviate the decrease of the number of erythrocytes and promote recovery from the decreased number of erythrocytes.

This was not previously known.

Accordingly, it now becomes possible to provide a new hemopoietic function-stimulating agent and pharmaceuticals and foods or beverages having hemopoietic function stimulating action, as well as pharmaceuticals and foods or beverages having an action to alleviate the decrease of the number of erythrocytes and to promote the recovery of the number of erythrocytes.

The present inventors carried out research and as a result, found that anserine, balenine, τ-methylhistidine and τ-methylhistidine exhibit prophylactic and recovery-accelerating effects on the decrease of hemopoietic function caused by drugs such as anticancer agents, by radiation etc., and that they have a stimulating effect on hemopoietic functions when the blood is lost.

In addition, the present inventors found that anserine, carnosine, balenine, homocarnosine, τ-methylhistidine and τ-methylhistidine exhibit an action to prevent and to promote curing of medical symptoms caused by a decrease in the number of erythrocytes.

Accordingly, in one aspect we provide a hemopoietic function-stimulating agent comprising at least one imidazol compound selected from the group consisting of anserine, balenine, τ-methylhistidine and τ-methylhistidine.

In another aspect we provide a food or a beverage having hemopoietic function stimulating action comprising at least one imidazol compound selected from the group consisting of anserine, balenine, τ-methylhistidine and τ-methylhistidine or an extract containing the same as a main ingredient.

In another aspect we provide a process for preparing food or a beverage having a hemopoietic function-stimulating action characterized by adding at least one imidazole compound selected from anserine, balenine, τ-methylhistidine and τ-methylhistidine, or an extract containing the same as a main ingredient, to a food or beverage.

In another aspect we provide a preventing or alleviating agent for medical symptoms caused by a decrease in the number of erythrocytes, comprising as an effective ingredient at least one imidazole compound selected from the group consisting of anserine, carnosine, balenine, homocarnosine, τ-methylhistidine and τ-methylhistidine.

In another aspect we provide foods or beverages having a preventing or alleviating action for medical symptoms caused by a decrease in the number of erythrocytes, comprising at least one imidazole compound selected from the group consisting of anserine, carnosine, balenine, homocarnosine, τ-methylhistidine and τ-methylhistidine, or an extract containing said imidazole compound as a main component.

### DETAILED DESCRIPTION

Anserine, balenine, τ-methylhistidine, τ-methylhistidine, carnosine and homocarnosine used in the present invention may be a product produced by extraction, isolation and purification from a natural product such as marine products broth discharged from the production process of boiled and dried fish such as bonitos or sardines, or canned tuna, or livestock meat such as chicken, those which are chemically or enzymatically synthesized, or those which are microbially produced. Anserine, balenine, τ-methylhistidine, τ-methylhistidine, carnosine and homocarnosine used in the present invention may be any of D-, L- and DL-forms. Note, since naturally occurring anserine, balenine, τ-methylhistidine, τ-methylhistidine, carnosine and homocarnosine are L-form, the L-form is preferable when they are used for foods or beverages.

Anserine, balenine, τ-methylhistidine and τ-methylhistidine, carnosine and homocarnosine used in the present invention may be a form of a salt; and salts of anserine, balenine, τ-methylhistidine, τ-methylhistidine, carnosine and homocarnosine may be salts based on the carboxy group, acid addition salts with pharmacologically acceptable acids based on the amino group, or salts based on both the carboxyl and amino groups.

The salts based on the carboxyl group include salts with metals such as sodium, potassium, calcium, magnesium, zinc and aluminum, ammonium salts as well as substituted ammonium salts such as salts with trialkylamine such as triethylamine or salts with other amines; the salts based on the amino group include salts with inorganic or organic acids such as hydrochloric acid, sulfuric acid, phosphor acid, acetic acid, propionic acid, lactic acid, tartaric acid, citric acid, succinic acid, maleic acid, benzenesulfonic acid, toluenesulfonic acid. These salts can be produced, according to known processes, by reacting free anserine, balenine, τ-methylhistidine, τ-methylhistidine, carnosine or homocarnosine with a stoichiometrically calculated amount of a selected base or acid.

The above-mentioned compounds can be used alone or in combination. In the present invention, anserine, balenine, τ-methylhistidine, τ-methylhistidine, carnosine and homocarnosine need not be very pure products, but an extract containing the imidazole compound as a main component can be used.

Here, the extract containing the present imidazole compound may be a marine products broth discharged from the production process of boiled and dried sardines or bonitos, or canned tuna, an extract extracted from livestock meat such as chicken according to a conventional procedure, or a fraction containing the present imidazole compound isolated using an ion exchange resin according to a conventional procedure. As an example for isolation and purification process, Japanese Unexamined Patent Publication (Kokai) No. 63-132878 titled Process for Isolation and Purification of Dipeptides from Bonito Broth can be mentioned.

Especially, a dipeptide fraction isolated and purified from a boiled liquid discharged during the production of canned tuna is preferable, because it contains abundant anserine. Note, the content of imidazole compound in an extract containing imidazole of the present imidazole compound(s) as main component should be at least 2%, preferably at least 10% and more preferably at least 50% while a content of anserine is at least 1%, preferably at least 5%, and more preferably 20%.

Anserine, balenine, τ-methylhistidine and τ-methylhistidine used in the present invention have actions to alleviate the decrease of hemopoietic function caused by drugs or radiation, or by stress, and accelerate the recovery from a reduced hemopoietic function. Accordingly, the compounds used in the present invention are useful as medicine or as a medical food aid for preventing or alleviating disorders of hemopoietic functions caused by drugs or radiation, by stress etc.

As disorders of hemopoietic function, there can be mentioned agranulocytosis, leukopenia, erythropenia, aplastic anemia, hemolytic anemia, megaloblastic anemia, pancytopenia, thrombocytopenia etc., and among them agranulocytosis is caused by drugs such as sulpha, anti-thyroidal agent, chloramphenicol, chlorothiazide, cimetidine, methyl DOPA, phenylbutazone and the others. Leukopenia, thrombocytopenia and erythropenia are caused by anticancer agents such as nitrogen mustard, busulfan, chlorambucil, cyclophosphamide, methotrexate, 6-mercaptopurine, 5-fluorouracil, actinomycin D, adreamycin, mytomycin C, procarvazine, cisplatin etc., and radiation.

Especially, the effective components used in the present invention are useful for treatment of leukopenia, thrombocytopenia, erythropenia and anemia caused by side effects of chemotherapeutic agents inhibiting the proliferation of cancer or viruses (anticancer agents, anti-AIDS agents), and leukopenia, thrombocytopenia, erythropenia and anemia caused by side effects of radiation. In addition, anserine, balenine, τ-methylhistidine and τ-methylhistidine have an activity to stimulate the hemopoietic function, and therefore the present compounds are useful as drugs or medical food aids for the stimulation of hemopoietic functions after an operation accompanied with bleeding, childbirth or blood donation.

In addition, anserine, carnosine, balenine, homocarnosine, τ-methylhistidine and τ-methylhistidine according to the present invention have an action to recover the number of erythrocytes by alleviating the decrease in the number of erythrocytes and by shortening the period of the decrease in the number of erythrocytes, and therefore are useful for pharmaceuticals or medical foods to prevent or alleviate medical symptoms caused by a decrease of the number of erythrocytes due to repression of hemopoietic function or loss of the blood.

The medical symptoms caused by a decrease in the number of erythrocytes, anemia by repression of hemopoietic function, anemia by loss of blood, aplastic anemia, congenial anemia, renal anemia, myelopathic anemia, anemia by chronic arthritic rheumatism etc.; the anemia by repression of hemopoietic function includes anemia caused by the decrease of the number of erythrocytes by chemotherapic agents such as anticancer agents, irradiation, stress etc.; and the anemia by loss of blood includes anemia caused by an operation or blood donation, or by childbirth.

Note that anemia is a condition wherein the number of erythrocytes or a content of hemoglobin in the blood absolutely decrease, and provides symptoms such as pale color of the skin or mucous membrane, pulsation, shortness of breath, giddiness, ears ring, fatigue, headache, chill, dropsy, taste-abnormality etc. Naturally, preventing or alleviating agents of the present invention for medical symptoms caused by the decrease of the number of erythrocytes are useful against the above-mentioned symptoms. According to the present invention, "alleviation" of the symptom is used in a wide sense, and includes therapy of diseases.

Where the present imidazole compound is used as a hemopoietic function-stimulating agent or a preventing or alleviating agent for medical symptoms caused by the decrease of the number of erythrocytes, any formulation which can be conveniently administered, orally or parenterally, may be accepted. For example there may be mentioned, for example, injections, infusions, powders, granules, tablets, capsules, enteric coated tablets, enteric capsules, suppositories, ointments, inhalants, troch and the like. These formulations can be used alone or in combination. These formulations can be produced according to a conventional procedure, depending on the purpose and using a principal drug and known aids conventionally used in the field of the production of pharmaceuticals such as excipient, binder, disintegrator, lubricant and corrigent.

The administration dose of the present hemopoietic function-stimulating agents or preventing or alleviating agent for medical symptoms caused by the decrease of the number of erythrocytes varies depending on administration route, formulation, condition, age, body weight etc., and usually for an adult, in case of oral administration, a total dose of the present effective components is 50 mg to 5 g/day, preferably 100 mg to 2 g/day, more preferably 50 mg to 2 g/day, more preferably 50 mg to 500 mg/day, still more preferably 50 mg to 180 mg/day, and still more preferably 50 mg to 100 mg/day. The dose of anserine or balenine is 50 mg to 5 g/day, preferably 100 mg to 2 g/day, more preferably 50 mg to 2 g/day, more preferably 50 mg to 500 mg/day, still more preferably 50 mg to 180 mg/day, and still more preferably 50 mg to 100 mg/day. The dose of τ-methylhistidine or τ-methylhistidine is 75 mg to 7.5 g/day, preferably 150 mg to 3 g/day, more preferably 75 mg to 3 g/day, and still more preferably 75 mg to 150 mg.

In case of parenteral administration, the total dose of the present effective components is 5 mg to 500 mg/day, preferably 10 mg to 200 mg/day, more preferably 5 mg to 200 mg/day, more preferably 5 mg to 50 mg/day, still more preferably 5 mg to 18 mg/day, and still more preferably 5 mg to 10 mg/day. The dose of anserine or balenine is 5 mg to 500 mg/day, preferably 10 mg to 200 mg/day, more preferably 5 mg to 200 mg/day, more preferably 5 mg to 50 mg/day, still more preferably 5 mg to 18 mg/day, and still more preferably 5 mg to 10 mg/day. The dose of τ-methylhistidine or τ-methylhistidine is 7.5 mg to 750 mg/day, preferably 15 mg to 300 mg/day, more preferably 7.5 mg to 300 mg/day, and still more preferably 7.5 mg to 15 mg/day.

Since the present anserine, balenine, τ-methylhistidine, τ-methylhistidine, carnosine and homocarnosine are originally, naturally occuring substances, their toxicity is low, and therefore they are significant as hemopoietic function-stimulating agents, or preventing or alleviating agents for medical symptoms caused by a decrease in the number of erythrocytes.

Where the present imidazole compound is used in foods or beverages having hemopoietic-function stimulating action, or preventing or alleviating action for medical symptoms caused by the decrease of the number of erythrocytes, although they can be formulated as described above, preferably the foods and beverages can be produced according to a general procedure by adding a necessary amount of the present effective component (anserine, balenine, τ-methylhistidine or τ-methylhistidine for hemopoietic-function stimulating agents; anserine, balenine, τ-methylhistidine, τ-methylhistidine, carnosine or homocarnosine for preventing or alleviating agent for medical symptoms caused by the decrease of the number of erythrocytes) or an extract containing the same as a main component to foods or beverages, especially those foods or beverages which originally substantially do not contain the present effective components. An amount thereof to be incorporated varies depending on formulations and the nature of the foods, and generally is preferably 0.001 to 50% though not limited thereto.

In the present invention, the foods or beverages substantially not including the effective components of the present invention are, for example, those not comprising meats such as beef, pork, chicken, fish meat, whale meat, snake meat and the like, as well as those not comprising an extract of meats such as beef, pork, chicken, fish meat, whale meat, snake meat or the like. However, foods and beverages made from meats such as beef, pork, chicken, fish meat, whale meat, snake meat etc., which contain a trace amount of anserine, balenine, τ-methylhistidine, τ-methylhistidine, carnosine or homocarnosine so that a total content of anserine, balenine, τ-methylhistidine, and τ-methylhistidine in a daily portion of the food is less than 50 mg, or a total content of anserine, balenine, τ-methylhistidine, τ-methylhistidine, carnosine and homocarnosine is less than 50 mg, or anserine in a meal is less than 50 mg are included in the present foods or beverages substantially not containing the present effective components.

For formulating foods or beverages, the present effective components or an extract containing the effective component as main components can be blended with proteins (as protein sources, milk protein, soybean protein, oligopeptide of egg white, hydrolysate of soybean protein, which have high nutrient value with good amino acid balance, or the like, or a mixture of amino acids may be used), sugars, fats together with trace elements, vitamins, emulsifier, perfume etc. to form a flowing food, a semi-flowing nutrient food or a nutrient component food. The foods or beverages containing the present effective components may be foods or grocery items in form of solid or liquid, for example, bread, noodles, rice, confectioneries (biscuit, cake, candy, chocolate, Japanese sweets), agricultural food products such as soybean curd and derivatives thereof, fermentation products such as Japanese sake or medical alcoholic drinks, sweet sake (mirin), vinegar, soysauce, livestock farming products such as soypaste, dressing, yogurt, ham, bacon, sausage, mayonnaise, fishery products such as steamed fish paste (kamaboko), fried fish paste, boiled fish paste (hanpen), or beverages such as fruit juice, soda drinks, sport drinks, alcoholic drinks, tea, and the like.

In addition for the purpose of prevention and alleviation of disorder of hemopoietic function such as leukopenia, thrombocytopenia etc. caused by drugs such as anticancer agents or radiation, or stimulation of hemopoietic function after operation, blood donation or childbirth, or for the purpose of prevention or alleviation of medical symptoms caused by the decrease of the number of erythrocytes, namely, for the purpose of prevention or alleviation of anemia by repression of hemopoietic functions such as anemia caused by chemotherapeutic agent such as anti-cancer agent or irradiation therapy, aplastic anemia, congenial anemia, renal anemia, myelopathic anemia, anemia by chronic arthritic rheumatism etc., or for the purpose for alleviation of anemia by loss of blood after operation, blood-donation or childbirth, under the control by a dietician according to a doctors diet prescription, it is possible to add powder of the present imidazole compound to any food during cooking in a hospital and to provide the in situ. cooked functional food to patients. Moreover the present foods or beverages can incorporate vitamins, hormones and other nutrients, and trace elements and iron-containing compounds.

For the purpose of prevention or improvement of various symptoms caused by insufficient hemopoietic function, or stimulation of hemopoietic function after operation, blood donation and childbirth, or for the purpose of prevention or alleviation of anemia caused by insufficient hemopoietic function or by a decrease of the number of erythrocytes by loss of blood etc., the present effective components are preferably orally administered in a total amount of the present effective components of at least 50 mg, preferably at least 100 mg, more preferably 50 mg to 5g, more preferably 100 mg to 2 g/day, more preferably 50 mg to 2g, more preferably 50 mg to 500 mg, still more preferably 50 mg to 180 mg, and still more preferably 50 mg to 100 mg per day. Anserine or balenine is orally administrated in an amount of at least 50 mg, preferably at least 100 mg, more preferably 50 mg to 5g, more preferably 100 mg to 2g, more preferably 50 mg to 2g, more preferably 50 mg to 500 mg, still more preferably 50 mg to 180 mg, and still more preferably 50 mg to 100 mg, per day. τ-methylhistidine or τ-methylhistidine is orally administrated in an amount of at least 75 mg, preferably at least 150 mg, more preferably 75 mg to 7.5g, more preferably 150 mg to 3g, more preferably 75 mg to 3g, and more preferably 75 mg to 150 mg, per day.

### EXAMPLE

The present invention is described more definitely by Examples, though the scope of the invention should not be limited to the Examples.

### Example 1. Spontaneous proliferation of bone marrow cells

300 mg/kg dose of cyclophosphamide (CY) was intraveously injected into 7-week-old ICR mice. 100 mg/kg of L-anserine (Ans) was subcutaneously injected every day from the day on which cyclophosphamide was injected. On the first, second, third, fifth, seventh and ninth day after administration of cyclophosphamide (on the third, fifth and seventh day for the group to which L-anserine was administrated), the femur of the mouse was aseptically removed, and both of the ends of the bone were cut off, and RPMI 1640 medium supplemented with 20% fetal calf serum (FCS) was poured into the cut end so as to recover bone marrow cells. The number of cells per femur for each mouse was counted, and the bone marrow cells for each test group were plated on a 96-well microplate (2 X 100,000 cells/well), and at the same time 0.5µ Ci/well of ³H-thymidine was added. The cells were cultured for 5 hours, and radioactivity (C.P.M) was measured with a liquid scintillation counter. An experiment was carried out using 3 animals per group, and the results are shown by mean value ± standard error. Spontaneous proliferation ability of bone marrow cells for each group was shown in the parentheses by percentage by taking spontaneous proliferation of bone marrow cells at 0 day (untreated) as 100%.

For the group to which CY alone was administered (the control group), the spontaneous proliferation ability of bone marrow cells which is an indication of hemopoietic ability was remarkably inhibited on first to third days from the administration, and on the fifth day the spontaneous proliferation ability was once recovered to the normal level, but on the seventh to ninth days it was again decreased. On the contrary, for the group to which L-anserine was administered together with CY, the bone marrow cells exhibited a spontaneous proliferation ability significantly higher than that of the control group on the third to seventh days from the administration, and especially on the third and seventh days, the proliferation ability at least two times higher than the control group was shown.

Therefore, it was demonstrated that L-anserine has spontaneous proliferation ability-stimulating action to bone marrow cells and stimulates the hemopoietic function.

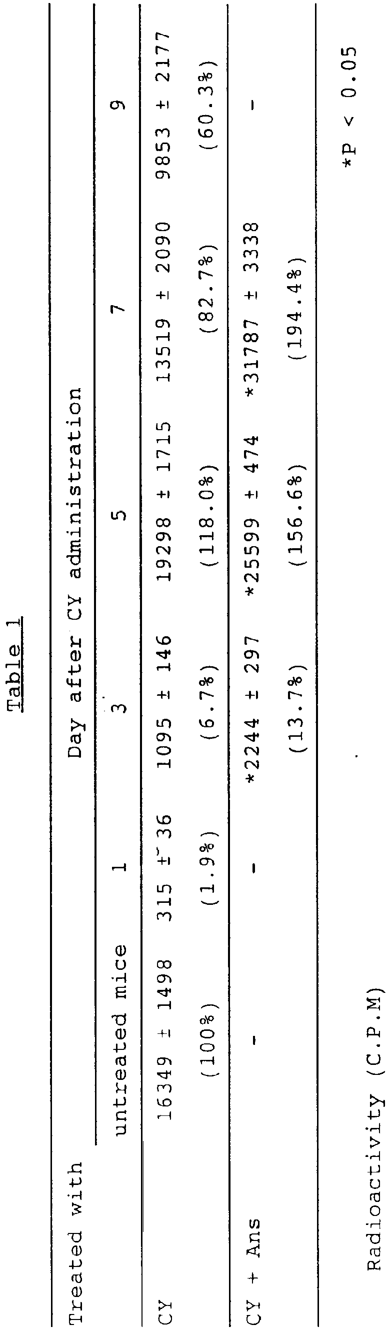

### Example 2. Colony formation-stimulating action in blood

300 mg/kg dose of cyclophosphamide (CY) was intravenously injected into 7-week-old ICR mice. 100 mg/kg dose of L-anserine (Ans) was subcutaneously injected every day from the day on which cyclophosphamide was administered. On the first, third, fifth, seventh and ninth days from the administration of cyclophosphamide (on the third, fifth and seventh day for the group to which Ans was administrated), the mouse serum was taken, and colony stimulating activity (CSA) was measured. For a CSA assay in serum, 50 µl of CSA assay serum was added to a medium comprising 50 µl of bone marrow cells (1.5 × 10⁶ cells/ml) aseptically recovered from the femur of a normal female ICR mouse, 200 µl of FCS, 450 µl of 0.6% agar and 300 µl of × 2 DMEM, and the mixture was stirred and cultured in 5% CO₂ at 37°C for 10 days, a cell mass comprising at least 30 cells was counted as one colony, and the number of the colonies was defined as the CSA of a serum. Three animals per group were used for experiment, and the results are shown as a mean value + standard deviation.

For the group to which CY alone was administered (the control group), on the first day of the administration, colony stimulating activity (CSA) in serum which is an indication of the hemopoietic function was inhibited, and after the third day the inhibition was compensated by stimulation of CSA. On the contrary, the group to which L-anserine was administered together with CY exhibited a CSA higher than that of the control on the third to seventh days from the administration, and especially on the third day a CSA 1.5 times higher than the control group was shown. Accordingly, it was demonstrated that L-anserine has a colony formation-stimulating action in serum, and stimulates the hemopoietic function.

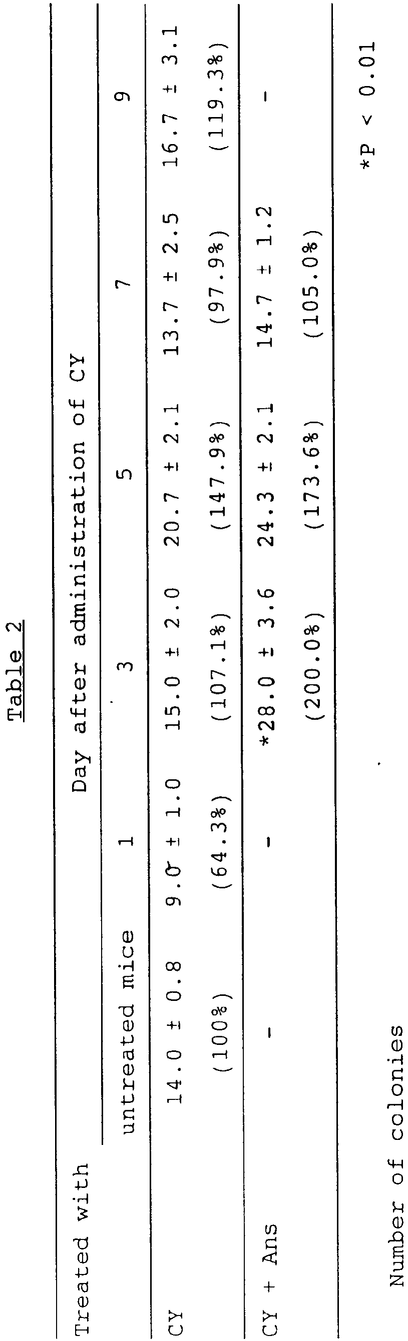

### Example 3. Hemopoietic function accelerating effects in spleen of mouse with cyclophosphamide

300 mg/kg dose of cyclophosphamide (CY) was intraveously injected to 7-week old ICR mice. To L-anserine (Ans)-administration group, 100 mg/kg dose of L-anserine was administrated every day from the day on which cyclophosphamide was administrated. On the third, fifth and seventh days from the administration of cyclophosphamide, spleen cells of the mouse were taken and the cells (5 × 10⁶ cells/ml) were cultured in a 10% FCS-RPMI 1640 medium supplemented with 2 µg/ml of concanavalin A for 24 hours, and the supernatant was assayed for IL-3. The IL-3 activity was measured by plating 1×10⁴ cells/well of IL-3 dependent FDCP-2 cells on a 96 well microtiter plate, adding twofold stepwise-diluted supernatant to the wells, culturing the cells for 24 hours, and measuring a ratio of surviving FDCP-2 cells by MTT assay using 3-(4,5-dimethylthyazol-2yl)-2,5-diphenyl tetrazolium bromide. Using a culture supernatant of WEHI cells as IL-3 standard, a concentration of IL-3 which provides 50% growth per the maximum growth of the cultured FDCP-2 cells was defined as 1 unit, and I L-3 activity of a sample was obtained by calculating a sample dilution ratio which provides 50% of the maximum absorption in the standard curve. Each experiment was carried out using three animals for one experiment, and the results are shown as a mean value ± standard error.

In the group to which CY alone was administrated (control group), the productivity of IL-3 by spleen cells, which is an indication of the hemopoietic function, was inhibited on the first to third days from the administration, and on and after the fifth days the inhibition was compensated by increase of IL-3 productivity by two times in comparison with the values of normal conditions. On the contrary, the group to which both CY and L-anserine were administered exhibited IL-3 productivity of the spleen cells higher than the control group on the third to seventh days of the administration, and especially it exhibited IL-3 productivity 1.5 times higher than the control group on the fifth and seventh days. Accordingly, it was demonstrated that L-anserine has IL-3 productivity stimulating action for the spleen cells, and stimulates the hemopoietic function.

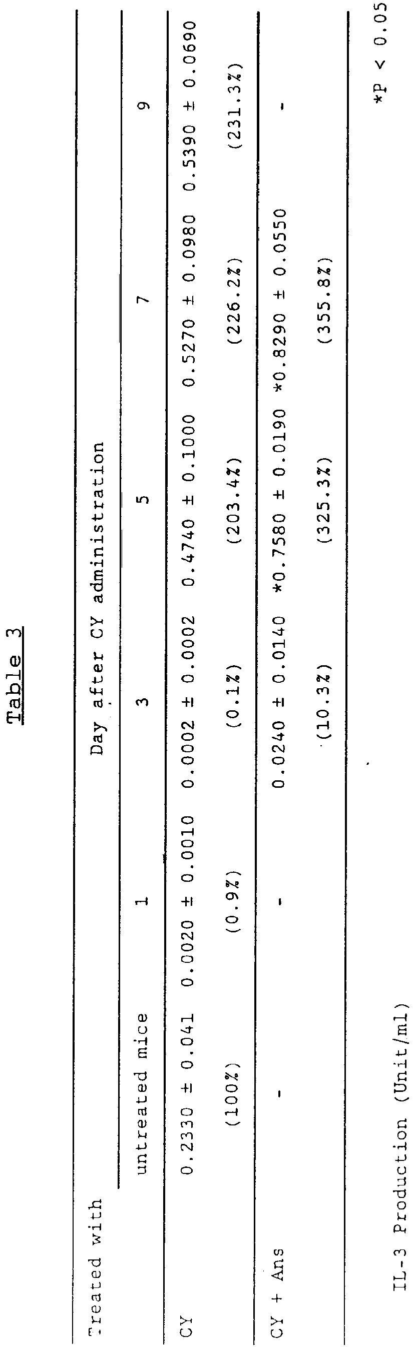

### Example 4. Hemopoietic function stimulating effect in spleen cells of mouse radiated with X-rays

Colony Forming Unit in Spleen (CFU-s) of mouse bone marrow cells in mouse radiated with X-ray was carried out using a transplant method. The X-ray radiation was carried out using an MBR-1520 type R X-ray radiation apparatus manufactured by Hitachi Medico. The mice were irradiated with X-rays such that the radiation dose rate was 1.16 Gy/min (150 kV, 20 mA, Filter: 0.5 mm Al + 0.1 mm Cu), and the radiation dose was 6 Gy. 5 × 10⁴ bone marrow cells of normal C57BL/6 mouse were injected into a tail vein of a radiated recipient female C57BL/6 mouse. Eight days later, the spleen of the mouse was removed and fixed with Bouin solution, and the number of cell colonies formed on the spleen surface were counted. To the L-anserine-administration group 100 mg/kg dose of L-anserine was subcutaneously administrated every day from the day on which X-ray was radiated to the seventh day, and to the chicken extract-administration group 0.5 ml/head dose of chicken extract containing 2mg/ml of L-anserine was orally administrated every day from the day on which X-ray was radiated to the seventh day. An experiment was carried out using eight animals per group, and the results are shown as a mean value ± standard error.

Colonies were not formed on the spleen of X-ray non-radiation group (normal mouse); and though colonies were formed in the X-ray radiation group (control group), CFU-s activity which is an indication that the hemopoietic function was detected. On the contrary, the L-anserine-administration group and the chicken extract-administration group showed CFU-s activity higher than that of the control group, and especially the L-anserine-administration group exhibited significantly higher CFU-s activity. Therefore, it was shown that L-anserine stimulates the hemopoietic function.

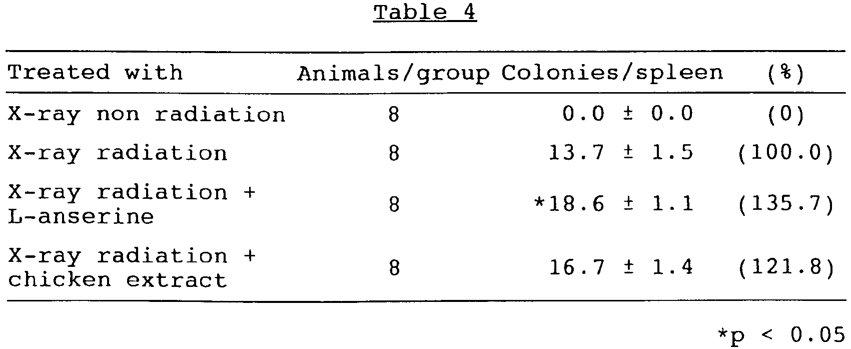

### Example 5. Leukocyte number recovery-stimulating effect in mouse with cyclophosphamide

300 mg/kg dose of cyclophosphamide (CY) was intravenously injected to 7-weeks old C57BL mice. 100 mg/kg dose of L-anserine (Ans) was subcutaneously administrated every day from the day on which cyclophosphamide was administered. On the seventh, eighth and ninth days from the administration of cyclophosphamide blood samples were taken from the orbital venosus plexus of the mouse, and the number of leukocytes was counted by an automatic hemacytometer. An experiment was carried out using 5 animals per group, and the results are shown as a mean value ± standard error.

For the CY alone-administration group (control group) the number of leukocytes, which is an indication of the hemopoietic function, decreased immediately after the administration, and gradually recovered before the seventh to ninth day. On the contrary, the group to which L-anserine was administered together with CY, showed the number of leukocytes higher than that of the control group on the seventh to the ninth days from the administration, and especially it showed the number of leukocytes almost 1.5 times higher than that of the control on the eighth and ninth days after the administration. Therefore, it was demonstrated that L-anserine has leukocyte number-recovery stimulating action and stimulates the hemopoietic function.

**Table 5**

| Treated with | Days after CY administration | | | |
|---|---|---|---|---|
| | untreated mice | 7 | 8 | 9 |
| CY | 87.4 ± 5.6 | 32.8 ± 8.0 | 67.4 ± 8.1 | 66.6 ± 7.2 |
| CY + Ans | - | 43.6 ± 3.1 | 108.2 ± 31.9 | 97.6 ± 20.5 |

The number of leukocytes (X 100/µl)

### Example 6. Effect of prevention of anemia in mouse administrated with the anticancer agent cyclophosphamide

7-week old C57BL-mice were intravenously injected with cyclophosphamide (CY) in a dose of 300 mg/kg. L-anserine (Ans) was subcutaneously administered every day in a dose of 100 mg/kg from the day on which cyclophosphamide was administered. After 1, 3, 5, 7 and 9 days from the administration of cyclophosphamide (for L-anserine, after 3, 5 and 7 days), blood samples were taken from the orbital venosus plexus of the mouse, and the number of erythrocyte and the amount of hemoglobin were measured by an automatic hemacytometer. The experiment was carried out using three mice per experimental group, and the results are shown as mean ± standard error. In the group to which CY was administered alone, during the third to seventh days from the administration, the number of erythrocytes and the amount of hemoglobin decrease, resulting in anemia. In contrast, in the group wherein CY and L-anserine were administered, the decreases of the number of erythrocytes and content of hemoglobin were inhibited during the third day to seventh day from the administration, revealing that L-anserine is effective to prevent anemia caused by an anticancer agent.

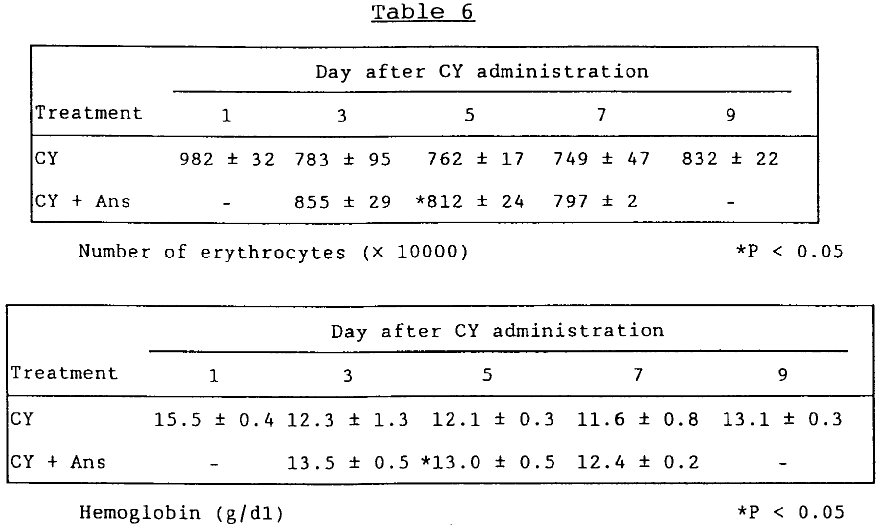

### Example 7. Effect of treatment of anemia in mice irradiated with X-ray

7-week old C57BL/mice were systemically irradiated with 4.0 Gy of X-ray. L-anserine was subcutaneously injected continuously for 10 days from the day on which X-ray was irradiated in a dose of 100 mg/kg. On 14th day from the irradiation of X-ray, blood samples were taken from the orbital venosus plexus of mice, and the number of erythrocytes and amount of hemoglobin were measured by an automatic hemacytometer. The experiment used three mice for one experimental group, and the results are shown as mean ± standard error. The number of erythrocytes and the content of hemoglobin decreased due to the X-ray irradiation were maintained at the decreased level on 14 day, while the group where L-anserine was administrated, the recovery of the number of erythrocytes and the concentration of hemoglobin were promoted, revealing that L-anserine is effective for therapy of anemia caused by X-ray irradiation.

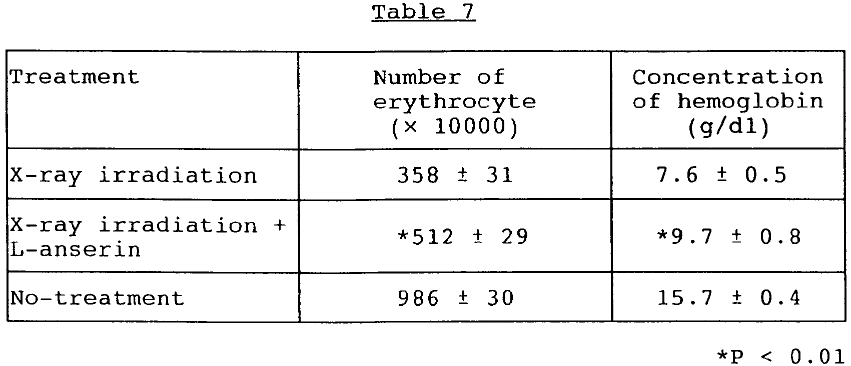

### Example 8.

A concentration of bonito broth was filtered though an ultrafilter with cut-off molecular weight 10,000 to obtain a clear low molecular weight fraction. The clear liquid was adjusted to a pH valve of 2.2 with diluted hydrochloric acid, and the resulting clean liquid was passed through a column filled with type MM 81 ionexchange resin from Beckman so as to adsorb anserine, and π-methylhistidine. After that, the column was eluted with 0.4N sodium citrate solution while keeping the column at 57°C so as to obtain a fraction containing anserine and π-methylhistidine. This fraction was lyophilized to obtain a dried product. This dried product was analyzed by an amino acid analyzer for the contents of anserine and π-methylhistidine. As a result, the contents of anserine and τ-methylhistidine were 56% and 8% respectively.

### Example 9.

A concentrate of a cooked extract discharged from the production process of canned tuna was filtered through an ultrafiltration membrane having a cut-off molecular weight of 10000 to obtain a clear low-molecular weight fraction. The clear fraction was adjusted to pH 2.2, and passed through a column filled with MM81 type ionexchange resin (Beckmann) to adsorb anserine and carnosine. Next, 0.4N sodium citrate solution was passed through the column to obtain a fraction containing anserine and carnosine. The fraction was lyophilized. The lyophilizate was subjected to an amino acid analysis. As a result the lyophilizate contained 41% anserine and 12% carnosine.

### Example 10.

Lactose 50 parts, sucrose 39.8 parts, tragacanth gum 5 parts, and peppermint 0.2 parts were mixed, and to the mixture were added anserine 5 parts, cupric chloride 0.002 parts and distilled water 3.5 parts, and the whole was thoroughly kneaded. Next, the kneaded product was put on a glass plate on which starch has been scattered, and was extended with a rolling bar so that the sheet of the kneaded product has thickness of about 5 mm. The sheet was shaped by stamping and dried to obtain troches.

### Example 11.

To wheat flour 100 parts were added carnosine 2 parts, manganese chloride 0.001 parts, sucrose 4 parts, salt 1.1 parts, skim milk 2 parts, yeast powder 3 parts, yeast food 0.8 parts and water 67.2 parts, and the whole was kneaded, and further fat 5 parts was added thereon, and the whole was thoroughly kneaded. The kneaded dough was subjected to a primary fermentation at 28°C for 90 minutes. Next, punching was carried out, and the dough was subjected to a secondary fermentation at 28°C, for 20 minutes, the mass was molded and baked at 190°C for 30 minutes. The properties of the baked bread was compared with that produced without adding anserine. As a result, there is no difference of the flavor and texture therebetween.

### Example 12.

To pork 80 parts were added anserine 1 parts, τ-methylhistidine 1 parts, cupric chloride 0.001 parts, sodium nitrite 0.02 parts, salt 2 parts, sodium pyrophosphate 0.3 parts, sodium ascorbate 0.06 parts and sucrose 1 parts and, after the meat was salted at 10°C for 72 hours, seasonings and spices were added thereon. The meat was cut to prepare kneaded meat. The kneaded meat was packed to a casing, and sausages were produced according to a conventional procedure. The sausage was subjected to a sensory test. As a result, the sausage thus produced was not different from that produced without adding anserine and τ-methylhistidine in their color, texture and flavor.

### Example 13.

Granules were produced according to the following recipe using synthetic anserine.

| | |
|---|---|
| Anserine | 0.2g |
| Cupric chloride | 0.02 mg |
| Lactose | 0.34g |
| Cornstarch | 0.45g |
| Hydroxypropylmethyl cellulose | 0.01g |

### Example 14.

To vitamin C 20g, ferrous sulfate 10g, granued sugar 40g, and an equal amount mixture of cornstarch and lactose 30g, was added 50g of an extract obtained in Example 8, and the whole was mixed. The mixture was divided 100 equal portions which were then packed in sacks. As a result, 100 packs of stick-shaped functional food weighting 1.5g and having hematopoietic function were produced.

## Claims

1. A hemopoietic function stimulating agent comprising at least one imidazole compound selected from the group consisting of anserine, balenine, τ-methylhistidine and τ-methylhistidine.

2. A food or beverage having a hemopoietic function stimulating action comprising at least one imidazole compound selected from the group consisting of anserine, balenine, τ-methylhistidine and τ-methylhistidine, or an extract containing the imidazole compound as a main component.

3. A food or beverage having hemopoietic function stimulating action, according to claim 2, comprising 50 mg to 5 g/day of at least one imidazole compound selected from the group consisting of anserine, balenine, π-methylhistidine and τ-methylhistidine.

4. A process for production of a food or beverage having hemopoietic function stimulating action, **characterized by** adding at least one imidazole compound selected from the group consisting of anserine, balenine, τ-methylhistidine and τ-methylhistidine, or an extract containing the imidazole compound as a main component to a food or beverage substantially not containing anserine, balenine, τ-methylhistidine and τ-methylhistidine.

5. A preventing or alleviating agent for medical symptoms caused by the decrease of the number of erythrocytes, comprising at least one imidazole compound selected from the group consisting of anserine, carnosine, balenine, homocarnosine, τ-methylhistidine and τ-methylhistidine.

6. Foods or beverages having an action to prevent or alleviate medical symptoms caused by the decrease of the number of erythrocytes, comprising at least one imidazole compound selected from the group consisting of anserine, carnosine, balenine, homocarnosine, τ-methylhistidine and τ-methylhistidine, or an extract containing the imidazole compound as a main component.

7. Foods or beverages according to claim 1, wherein the medical symptoms caused by the decrease of the number of erythrocytes is anemia by repression of hemopoietic function or loss of blood.

8. Use of anserine, balenine, n-methylhistidine or τ-methylhistidine in the preparation of a medicament for the treatment of illness consequent upon disorder of the hemopoietic function.

9. Use according to claim 8 in which the illness is agranulocytosis, leukopenia, erythropenia, anemia, pancytopenia, thrombocytopenia or blood loss.

10. Use of anserine, carnosine, balesine, homocarnosine, τ-methylhistidine or τ-methylhistidine in the preparation of a medicament for the treatment of illness consequent upon a decrease in the number of erythrocytes.

11. Use according to claim 10 in which the illness is an anemia.
